Europäisches Patentamt

European Patent Office

Office européen des brevets

(11) Veröffentlichungsnummer: **0 366 968**

**A1**

## (12) EUROPÄISCHE PATENTANMELDUNG

(21) Anmeldenummer: **89118455.8**

(22) Anmeldetag: **05.10.89**

(51) Int. Cl.⁵: **C08L 35/00 , C08L 29/02 , C08L 1/00 , C08L 3/00 , C08L 5/00 , A61L 15/24 , A61L 15/28 , A61L 15/60**

(30) Priorität: **14.10.88 AT 2559/88**

(43) Veröffentlichungstag der Anmeldung:
**09.05.90 Patentblatt 90/19**

(84) Benannte Vertragsstaaten:
**AT BE CH DE ES FR GB IT LI LU NL SE**

(71) Anmelder: **Chemie Linz Gesellschaft m.b.H.
St.Peter-Strasse 25
A-4021 Linz(AT)**

(72) Erfinder: **Häubl, Georg, Dipl.-Ing. Dr.
Mitterleitenweg 12c
A-4040 Linz(AT)**
Erfinder: **Scheuchenstuhl, Willibald
Neudorf 22
A-4271 St. Oswald(AT)**

(74) Vertreter: **Kunz, Ekkehard, Dr.
Chemie Holding AG Patentwesen St.
Peter-Strasse 25
A-4021 Linz(AT)**

(54) Absorbierendes Polymerisat.

(57) Absorbierendes Polymerisat, bestehend aus einem Polymer A, das ein gegebenenfalls modifizierter Polyvinylalkohol oder ein gegebenenfalls modifiziertes Polysaccharid oder eine Mischung derselben ist und aus einem Polymer B, das ein Maleinsäureanhydrid-, Homo- oder Copolymer mit einer Hydrolysezeit von 5 bis 120 Minuten ist, wobei die Anhydridgruppen geöffnet und 0,005 bis 5 Mol-% der Säurefunktionen mit Hydroxygruppen des Polymers A verestert sind und die übrigen Säurefunktionen als freie Säuregruppen, als Salze oder als Säureamidgruppen vorliegen, ein dieses enthaltendes absorbierendes Erzeugnis und Verfahren zu deren Herstellung.

EP 0 366 968 A1

## Absorbierendes Polymerisat

Die Erfindung betrifft ein neues, absorbierendes Polymerisat und ein absorbierende Erzeugnis, das dieses enthält, Verfahren zu deren Herstellung und deren Verwendung zur Aufnahme und zum Zurückhalten von Wasser oder wäßrigen Lösungen.

Absorbierende Polymerisate bestehen im allgemeinen aus Polymeren oder Copolymeren, die hydrophil sind und die durch Reaktion mit geeigneten Vernetzern wasserunlöslich gemacht wurden. Solche Materialien können Flüssigkeiten oder Wasserdampf absorbieren und festhalten, sodaß sie breiteste Anwendung auf verschiedensten Gebieten, insbesondere aber im sanitären Bereich finden. In Verbindung mit einem Träger werden sie überall dort, wo schnell Flüssigkeiten entfernt werden sollen, also beispielsweise in Küchenrollen, Windeln, Tampons, Bandagen usw. eingesetzt. Andererseits können solche Polymerisate mit gutem Erfolg auch zum Zurückhalten und späteren Abgeben von Feuchtigkeit auf landwirtschaftlichem Sektor aber auch in vielen anderen Bereichen angewendet werden. Es hat daher bisher nicht an Versuchen gefehlt, solche Polymerisate bereitzustellen.

So ist in der DE-A-29 23 435 ein Verfahren zur Herstellung von vernetztem Polyvinylalkohol (PVAL), in der DE-A-25 20 337 ein Verfahren zur Herstellung von vernetzter Cellulose, in der DE-A-28 23 710 ein Verfahren zur Herstellung vernetzter Carboxyalkylcellulose und in GB-A-1 508 123 ein Verfahren zu Herstellung vernetzter Stärke mit absorbierenden Eigenschaften geoffenbart. Abgesehen davon, daß diese vernetzten, hydrophilen Polymer oft einen hohen Anteil wasserlöslicher Produkte enthalten, werden als Vernetzungsmittel giftige epoxid-, halogen- oder acrylgruppenhaltige Monomere eingesetzt. Da die vernetzten, polymeren Materialien immer in einem gewissen Ausmaß noch unreagierte, monomere Vernetzer enthalten, kann insbesondere deren Anwendung im sanitären Bereich gesundheitsschädigende Wirkungen hervorrufen.

US-A-4,332,917 beschreibt die Herstellung einer Polymerlegierung aus einem MSA-Styrol-Copolymeren und einen Celluloseester in einem organischen Lösungsmittel. Dazu werden die Maleinsäureanhydridgruppen zumindest teilweise verseift, die beiden Polymere in einem organischen Lösungsmittel vermischt und das Lösungsmittel verdampft. In EP-A-0 210 754 wird eine Zusammensetzung von Polyalkylenoxiden mit Copolymeren, die aus Einheiten eines alpha-beta-ungesättigten Monomers und eines damit copolymerisierbaren Monomers bestehen, zur Herstellung einer wasserabsorbierenden Zusammensetzung beschrieben. In beiden Fällen werden jedoch die jeweiligen Polymere nur durch Wasserstoffbrücken zusammengehalten.

Aus DE-A-22 28 256 ist bekannt, Polyethylenoxid mit anderen wasserlöslichen Polymerisaten mit Hilfe ionisierender Strahlung zu vernetzen. Anlagen, die die Bestrahlung von Substraten mit ionisierender Strahlung ermöglichen sind aber technisch aufwendig und erfordern umfangreiche Schutzmaßnahmen.

Mit Hilfe der vorliegenden Erfindung ist es unerwarteterweise gelungen, absorbierende Polymerisate herzustellen, die das Nachteile, die die oben angeführten Polymerisate und deren Herstellung besitzen, überwindet.

Gegenstand der Erfindung ist demnach ein absorbierendes Polymerisat, bestehend aus einem Polymer A und einem Polymer B, dadurch gekennzeichnet, daß das Polymer A zu einem Anteil von 10 bis 99 Gewichtsprozent vorliegt und ein gegebenenfalls modifizierter Polyvinylalkohol oder ein gegebenenfalls modifiziertes Polysaccharid oder eine Mischung derselben ist und das Polymer B zu einem Anteil von 1 bis 90 Gewichtsprozent vorliegt und ein Maleinsäureanhydrid, Homo- oder Copolymer mit einer Hydrolysezeit von 5 bis 120 Minuten ist, wobei die Anhydridgruppen geöffnet und 0,005 bis 5 % der Säurefunktionen mit Hydroxygruppen des Polymers A verestert sind und die übrigen Säurefunktionen als freie Säuregruppen, als Salze oder als Säureamidgruppen vorliegen.

Polymer A ist ein gegebenenfalls modifizierter Polyvinylalkohol (PVAL) oder ein gegebenenfalls modifiziertes Polysaccharid oder eine Mischung solcher Polymeren. Unter PVAL sind übliche Polyvinylalkohole mit Molekulargewichten von etwa 10 000 bis 100 000 zu verstehen. Modifizierter PVAL bedeutet, daß ein Teil der Hydroxygruppen des PVAL verestert oder verethert vorliegt. Bei verestertem PVAL handelt es sich entweder um teilverseiftes Polyvinylacetat, oder um ganz oder teilweise verseiftes Polyvinylacetat, das neuerlich verestert wird. Verbindungen, die zur Veresterung von PVAL geeignet sind, sind z. B. reaktive Derivate von Di-oder Tricarbonsäuren wie z. B. Säureanhydride von Bernsteinsäure, Maleinsäure, Fumarsäure, Itaconsäure, Pyromellithsäure. Der Substitutionsgrad DS beträgt nach der Veresterung 0,005 bis 0,5, bevorzugt 0,01 bis 0,3. Solche Produkte sind käuflich erwerbbar oder sie können durch eine übliche Veresterungsmethode, etwa mit Hilfe eines reaktiven Carbonsäurederivatives gegebenenfalls in einem Verdünnungsmittel unter Verwendung basischer oder saurer Katalysatoren hergestellt werden. Verbindungen, die zur Veretherung geeignet sind, sind aliphatische oder araliphatische Sulfonsäuren, Phosphonsäuren, Carbonsäuren oder deren Salze, sowie quaternäre Ammoniumsalze mit einer aliphatischen Seitenkette,

2

wobei diese Seitenkette eine reaktive Abgangsgruppe oder eine Epoxidgruppe trägt und gegebenenfalls noch durch Hydroxygruppen substituiert sein kann. Beispiele für solche Verbindungen sind etwa 3-Chlor-2-hydroxypropansulfonsäure-Na, Chlormethylsulfonsäure, Chlormethylphosphonsäure-Na, Monochloressigsäure-Na, Glycidyltrimethylammoniumchlorid. Der Substitutionsgrad DS beträgt nach der Veretherung 0,005 bis 0,5 , bevorzugt 0,01 bis 0,3. Solche Produkte sind käuflich erwerbbar oder sie können unter üblichen Veretherungsbedingungen hergestellt werden, wobei die reaktive Abgangsgruppe aus der zur Veretherung eingesetzten Verbindung und als Wasserstoffion aus der Hydroxygruppe des PVAL gegebenenfalls in einem Lösungsmittel in Gegenwart einer Base abgespalten wird, wodurch eine Etherbindung ausgebildet wird. Im Falle, daß anstatt einer reaktiven Abgangsgruppe eine Epoxidgruppe vorliegt, erfolgt die Veretherung unter basischer Katalyse, wobei nach der Veretherungsreaktion in alpha-Stellung zum Ethersauerstoff auch noch eine Hydroxygruppe in der aliphatischen Seitenkette der zur Veretherung verwendeten Verbindung vorliegt. Substanzen, die zur Veresterung oder Veretherung Verwendung finden, sind bekannte Substanzen der organischen Chemie. Bevorzugt ist Polymer A ein modifizierter PVAL, beispielsweise in durch Veresterung mit Bernsteinsäureanhydrid modifizierter PVAL.

Als Polysaccharide kommen beispielsweise Stärke, Dextran, Xanthan, wasserlösliche Cellulosederivate wie z. B. Celluloseether wie etwa Hydroxyalkylcellulose Methylcellulose, Carboxymethylcellulose, Hydroxypropylcellulose, Natriumalginat, Guar und ähnliche Stoffe in Betracht. Modifizierte Polysaccharide sind Polysaccharide, in denen ein Teil der Hydroxygruppen mit Verbindungen wie sie oben für PVAL beschrieben sind, verestert oder verethert sind. Modifizierte Polysaccharide sind bekannt und käuflich zu erwerben, oder sie können, wie oben für die modifizierten PVALe beschrieben, hergestellt werden. Bevorzugte Polysaccharide sind Carboxymethylcellulose oder Natriumalganit. Polymer A kann aber auch eine Mischung der oben beschriebenen Polymere sein. Bevorzugte Mischungen sind etwa teilverseifte PVAL und Carboxymethylcellulose oder teilverseifter PVAL und Natriumalginat. Bevorzugt ist Polymer A wasserlöslich.

Polymer B ist ein Maleinsäureanhydrid (MSA)-Homo- oder Copolymer oder eine Mischung aus MSA-Homo- und -Copolymer oder eine Mischung verschiedener MSA-Copolymere, wobei die Hydrolysezeit 5 bis 120 Minuten, vorzugsweise 10 bis 60 Minuten beträgt. Unter Hydrolysezeit ist jene Zeit in Minuten zu verstehen, in der der pH-Wert einer gerührten Dispersion von 0,2 g Polymer B in 76 ml destilliertem Wasser und 17,5 ml 0,1 molarer NaOH bei 25 °C von pH = 12,4 auf pH = 10,0 absinkt. MSA-Homopolymere sind bekannt und können beispielsweise nach EP-A-0 263 939 hergestellt werden. Copolymere von MSA mit Alkylvinylether, Styrol, 2-Methylstyrol, Monomethoxypolyethylenglykolvinylether oder Olefine, wie Ethylen, Propylen, Isobutylen, etc. und ähnliche Verbindungen, finden bevorzugt Verwendung. Besonders bevorzugt sind Maleinsäureanhydrid-Methylvinylether (MSA-MVE)-Copolymere. Copolymere von MSA mit obgenannten Verbindungen sind bekannt oder sie können durch übliche Methoden, MSA-Monomethoxypolyethylenglykolvinylether-Copolymer etwa nach Tohru Suzuki et al, Journal of Polymer Science: Polymer Chemistry Edition, Vol. 22, 1984, 2829 bis 2839, hergestellt werden. Bevorzugt ist Polymer B wasserunlöslich.

Die Säureanhydridgruppen liegen im erfindungsgemäßen absorbierenden Polymerisat praktisch vollständig geöffnet und abhängig von der Art und Menge der verwendeten Base zu einem geringen, aber wesentlichen Teil als Estergruppen, zum Teil als Salz und freie Säuregruppen, und in manchen Fällen auch als Säureamidgruppen vor. Der Neutralisationsgrad ist jener Anteil der Säurefunktionen in Mol-% bezogen auf die Gesamtanzahl der vorhandenen Säurefunktionen, der als Salz vorliegt. Er beträgt etwa 25 bis 80 %.

Der Anteil des Polymer A im erfindungsgemäßen absorbierenden Polymerisat beträgt 10 bis 99, bevorzugt von 40 bis 95 Gew.%, der Anteil des Polymer B von 1 bis 90, bevorzugt 25 bis 60 Gew.%.

Das erfindungsgemäße Polymerisat weist ein Absorptionsvermögen für destilliertes Wasser von mindestens 110 bis 800 g/g trockenes Polymerisat, ein Absorptionsvermögen für eine 0,9%ige wäßrige NaCl-Lösung oder für eine wäßrige CIPAC-D-Standardlösung, das ist eine wäßrige Lösung mit einem definierten Ionengehalt, die im Beispiel 1 der vorliegenden Anmeldung beschrieben ist, von 10 bis 120 g/g trockenes Polymerisat auf. Es entfaltet seine Absorptionsfähigkeit über einen weiten Temperaturbereich, der den üblichen Anwendungsbereich von etwa 0 bis 50 °C umfaßt und ist gegen die Flüssigkeiten, die es absorbieren soll, die neben Wasser z B. auch Blut, Urin, Schweiß, Wundsekrete usw. sein können, beständig. Ein besonderer Vorteil liegt in seiner Ungiftigkeit.

Gegenstand der Erfindung ist auch ein Verfahren zur Herstellung eines absorbierenden Polymerisates, daß dadurch gekennzeichnet ist, daß man einen gegebenenfalls modifizierten Polyvinylakohol, ein gegebenenfalls modifiziertes Polysaccharid oder Mischungen derselben mit einem Maleinsäureanhydrid-Homo-oder Copolymer mit einer Hydrolysezeit von 5 bis 120 Minuten vereinigt und bei einer Temperatur von 20 bis 120 °C in einem Guß mit 0,5 bis 1,6 Äquivalenten einer anorganischen oder organischen Base pro Äquivalent Maleinsäureanhydrid versetzt.

Zur Herstellung des erfindungsgemäßen absorbierenden Polymerisats, die kontinuierlich oder diskonti-

nüierlich erfolgen kann, werden zuerst Polymer A und Polymer B in fester, gelöster oder dispergierter Form vermischt. Als Verdünnungs- oder Lösungsmittel eignen sich unter den Reaktionsbedingungen inerte organische Lösungsmittel oder Wasser, wobei Wasser bevorzugt ist. Bevorzugt wird Polymer A in Wasser gelöst und Polymer B in Pulverform oder in Wasser dispergiert zugegeben. Die beiden Polymersysteme werden vereinigt und gegebenenfalls erhitzt, oder man erhitzt die Polymersysteme A bzw. B noch vor der Vereinigung. Die Reaktionsmischung wird bei Temperturen von 20 bis 120 °C, bevorzugt bei Temperaturen von etwa 50 bis 90 °C mit einer anorganischen oder organischen Base versetzt. Als anorganische Base können z. B. Alkalihydroxide, - hydrogencarbonate, -carbonate, Ammoniak, Ammoniumhydrogencarbonat, Ammoniumcarbonat, als organische Basen z. B. Amine wie Methylamin, Ethylamin, Guanidincarbonat usw. eingesetzt werden. Die Base wird bevorzugt in Form wäßriger Lösungen eingesetzt. Es ist wesentlich für das Verfahren, daß die Base in einem Guß, daß heißt schnell und ohne Unterbrechung zugesetzt wird.

Im allgemeinen werden die Lösungen bzw. Dispersionen der Polymere A und B so konzentriert als möglich eingesetzt.

Bei Zugabe der Base zu der Reaktionsmischung der Polymeren A und B, werden praktisch alle Maleinsäureanhydridgruppen des Polymers B geöffnet, wobei 0.005 bis 5 Mol-% der durch die Reaktion entstehenden Säurefunktionen eine Esterbindung mit den Hydroxygruppen des Polymers A eingehen. Dadurch entsteht ein kovalent verbundenes Netzwerk der Polymeren A und B mit einem geringen aber wesentlichen Anteil an kovalenten Esterbindungen. Die Anzahl der kovalenten Esterbindungen im absorbierenden Polymerisat wurde über das Dinitrophenylhydrazid, das bei 340 nm absorbiert, photometrisch bestimmt. Die Säurefunktionen liegen nach der Reaktion teils als Salz und teils als freie Säure vor, werden Ammoniak oder Amine verwendet, können darüber hinaus auch noch Amidbindungen entstehen.

Polymer A und Polymer B werden in einem Gewichtsverhältnis von 10 : 90 bis 99 : 1, bevorzugt von etwa 40 : 60 bis etwa 95 : 5 eingesetzt. Pro Äquivalent Maleinsäureanhydrid im Polymer B werden 0,5 bis 1,6 Äquivalente der Base verwendet, sodaß nach der Reaktion 25 bis 80 % der Säurefunktionen als Salz und 0 bis 50 % als Amid vorliegen. Die übrigen Säurefunktionen, die nicht als Ester, Amid oder Salz vorliegen, liegen als freie Säuregruppen vor.

Die Reaktion findet in sehr kurzer Zeit statt. Die Reaktionsgeschwindigkeit ist abhängig von der Art und Konzentration der jeweils eingesetzten Ausgangsstoffe, insbesonders aber von der Reaktionstemperatur. Bei Raumtemperatur verläuft die Reaktion innerhalb von Minuten, bei höheren Temperaturen innerhalb von Sekunden.

Im Verlauf der Reaktion steigt die Viskosität der Reaktionsmischung stark an. Daß dies zum Teil auf eine "Verknäuelung" der Polymeren A und B, wie in US-A-4,169.818 beschrieben, als wesentliches Element jedoch auch auf die geringfügige Veresterung von Polymer A und B zurückzuführen ist, wurde in Vergleichsversuchen festgestellt. Dazu wurden unter identischen Bedingungen jeweils erfindungsgemäße Reaktionsmischungen und Vergleichs-Reaktionsmischungen hergestellt. Der einzige Unterschied zwischen diesen Reaktionsmischungen bestand darin, daß im Vergleichsfall die Anhydridgruppen des Polymers B schon vor Zugabe der Base vollständig verseift waren, sodaß eine Veresterung mit Polymer A nicht mehr möglich war. Beim Vergleich der Viskositäten zeigt sich, daß die Viskosität der erfindungsgemäßen Reaktionsmischungen ganz wesentlich über der Viskosität der Vergleichs-Reaktionsmischungen liegt. Dies kann nur durch Vorliegen von Esterbindungen zwischen Polymer A und Polymer B erklärt werden.

Das bei der Reaktion entstandene Gel wird zur Gewinnung eines getrockneten absorbierenden Polymerisates beispielsweise auf eine geeignete Trägeroberfläche, wie etwa Glas, Kunststoff oder Stahl aufgebracht, durch übliche Methoden wie etwa Erhitzen, Trocknen an der Luft, Vakuumtrocknen, Lyophilisieren getrocknet , von der Trägeroberfläche abgezogen und als Folie verwendet oder abgekratzt und durch Verreiben oder Vermahlen verkleinert, sodaß das absorbierende Polymerisat anschließend in Film-, Pulveroder Flockenform vorliegt und verwendet werden kann.

Dem getrockneten Polymerisat oder einer Dispersion oder Lösung des erfindungsgemäßen Polymerisates können je nach Anwendungsgebiet verschiedene Stoffe wie Weichmacher, oberflächenaktive Stoffe, Füllstoffe, Pigmente, UV-absorbierende Materialien, Antioxydantien, Riechstoffe, Desinfektionsmittel oder auch für die Agrikultur geeignete Chemikalien zugemischt werden, sofern sie die Absorptionseigenschaften des erfindungsgemäßen Polymerisates nicht negativ beeinflussen.

Das erfindungsgemäße Polymerisat ist leicht und schnell herzustellen, ist praktisch wasserunlöslich, ungiftig, zeigt sehr gute Absorptionseigenschaften und stellt somit eine Bereicherung der Technik dar. Dementsprechend findet das erfindungsgemäße Polymerisat zahlreiche Anwendungsmöglichkeiten in Pulver-oder Stückform, in Form von Folien, Fasern, Flächengebilden und ähnlichen Formen. Folien, die aus dem erfindungsgemäßen Polymerisat hergestellt sind können z. B. benutzt werden, um Feuchtigkeitssperren im Erdboden zu errichten. Pulver, das aus dem erfindungsgemäßen Polymerisat besteht und das etwa mit Erde, Glasperlen, geschäumten Polymeren, calciniertem Ton oder zerkleinertem Kunststoff vermischt werden kann, verbessert die Wasserrückhalteeigenschaften des Bodens. Man kann auch Wirkstoffe auf irgendeine übliche Weise in das erfindungsgemäße Polymerisat einbringen, wodurch eine lang andauernde Wirkung dieser Wirkstoffe gewährleistet ist.

Weiters kann des absorbierende Polymerisat mit einem oder mehreren Träger zu einem absorbierenden Erzeugnis verbunden sein.

Als Träger kommen beispielsweise faserige Träger wie etwa gewebtes oder ungewebte Material wie z. B. Baumwollgewebe, Rayon, Wolle, Verbandgaze, Papier oder Celluloseflusen, z. B. in Form von Bahnen, Blättern oder losen Fasern, bevorzugt Papierbahnen in Betracht; in Frage kommt auch Glas, Keramik oder Metall, aber auch Materialien wie Holz, Stein oder Zement.

Das Polymerisat kann auf den Träger ein- oder beidseitig aufgebracht, zwischen mehreren gleichen oder auch verschiedenen Trägern ein-und oder aufgebracht sein, sodaß Laminate mit zwei oder mehr Schichten des Polymerisates vorliegen, oder es liegt in Verbindung mit losen Fasern wie z. B. Cellulosefasern, Asbestfasern oder anderem Material vor, wobei es zwischen Deckblättern aus beispielsweise Stoff, Vliesstoff oder Papier eingeschlossen sein kann. Es kann auf den Träger kontinuierlich oder diskontinuierlich, das heißt in Form von etwa Streifen, Punkten, Gittern usw. aufgebracht oder zwischen 2 oder mehrere Träger eingebracht sein.

Bevorzugte Erzeugnisse sind beispielsweise Erzeugnisse, die im Sanitärbereich Verwendung finden, wie etwa Haushalts- und Industrietücher, z. B. Küchenrollen, Monatsbinden, Tampons, chirurgische Schwämme oder Tupfer, Gesichtstücher, Bandagen, Binden usw.

Die Herstellung solcher absorbierenden Erzeugnisse kann auf verschiedene Art und Weise erfolgen. Der Träger kann wie üblich in eine Dispersion des erfindungsgemäßen Polymerisates getaucht und getrocknet werden, die Dispersion kann auf den oder die Träger aufgesprüht werden, oder das absorbierende Polymerisat wird in Pulverform auf den Träger aufgestreut und durch geeignete Maßnahmen wie etwa Behandeln mit Dampf und/oder Druck mit dem Träger adhesiv verbunden. Zum Beschichten von Fasern können in eine Faseraufschlämmung Polymer A, Polymer B und in einem Guß die Base eingebracht werden, sodaß das absorbierende Erzeugnis direkt in der Reaktionsmischung entsteht. Weiters ist es möglich, auf eine sich kontinuierliche fortbewegende Trägerbahn Polymer A, Polymer B sowie die Base in Lösung oder Disperion aufzusprühen. Dabei können Polymer A, Polymer B und die Base jedes für sich oder vereint aufgesprüht werden.

In einer besonders bevorzugten Ausführungsform wird eine wäßrige Lösung des Polymers A, mit dem Polymer B, das als Pulver oder in wäßriger Dispersion eingesetzt wird, vereinigt. Diese Dispersion und die Lösung der Base, die beide möglichst konzentriert sind, werden mit Hilfe von Preßluft oder Wasserdampf durch Düsen getrieben und auf den Träger aufgesprüht. Die Vermischung der Base mit Polymer A und Polymer B kann dabei noch in der Düse, oder außerhalb der Düse erfolgen. Wird als Träger eine Papierbahn verwendet, und wird die Reaktionsmischung sehr konzentriert und nur in geringen Mengen aufgetragen, ist es möglich, daß ein insbesondere übertrocknetes Papier die gesamte Feuchtigkeit aufnimmt.

Enthält der Träger freie Hydroxygruppen, wie etwa bei cellulosehältigen Trägern, können diese in den Vernetzungsprozeß involviert werden, sodaß bei der Ausbildung der absorbierenden Schicht auch noch eine besonders gute Verbindung mit dem Träger erfolgt.

Die absorbierenden Erzeugnisse zeigen ausgezeichnete Absorptionseigenschaften, wobei die Verbindung zwischen Träger und Polymerisat gut und dauerhaft ist, sie sind einfach, schnell und mit geringem Energie- und Kostenaufwand herzustellen und stellen somit eine Bereicherung der Technik dar.

Beispiel 1

Maleinsäureanhydrid-Methylvinylether (MSA-MVE-Copolymer)

In einem 2 l Juchheim Raktionsdruckbehälter wurden 87,3 g Maleinsäureanhydrid und 1,46 g Dilauroylperoxid vorgelegt, worauf die Reaktionsapparatur mit Stickstoff gespült wurde. Nachdem die Apparatur sauerstofffrei war, wurden 310 g an Methylvinylether der über festes Natriumhydroxid getrocknet und frisch destilliert war, zugegeben. Das Reaktionsgemisch wurde unter Rühren auf 55 °C erwärmt und 2 Stunden unter Druck auf dieser Temperatur gehalten. Nach Abkühlen auf 20 °C wurde der überschüssige Methylvinylether abdestilliert und das Reaktionsprodukt aus dem Behälter entnommen. Dabei entstanden 118 g (84,9 % der Theorie) eines MSA-MVE-Copolymers mit folgenden Eigenschaften: Trockenverlust bei 105 °C: 0,92 %, bei 60 °C im Vakuum: 0,25 %. Die Korngrößenverteilung, die durch Siebanalyse

5

gemessen wurde, ergab folgende Werte:

| Korngröße (Mikrometer) | (%) |
|---|---|
| unter 32 | 43,6 |
| 32 - 44 | 7,6 |
| 44 - 100 | 13,0 |
| 100 - 200 | 14,0 |
| 200 - 354 | 14,2 |
| 354 - 500 | 5,8 |
| 500 - 710 | 0,6 |
| 710 - 850 | 0,6 |
| 850 - 1400 | 0,6 |

Bedingungen: Alpine Luftstrahlsieb, 0,02 bar Unterdruck; Siebdauer: 7 Minuten.

Die spez. Viskosität wurde folgendermaßen bestimmt: 0,5 g MSA-MVE-Copolymer wurde in 50 ml Methylethylketon gelöst, 1 bis 2 Stunden bei Raumtemperatur gerührt. Die spez. Viskosität wurde mit Hilfe eines Ubbelohde-Viskosimeters gemessen und betrug 3,2.

Die Hydrolysegeschwindigkeit der MSA-Gruppen wurde auf folgende Weise bestimmt:

17,5 ml einer 0,1 molaren wäßrigen Natriumhydroxidlösung und 76 ml destilliertes Wasser (mit einem pH-Wert von 12,4) wurden mit 0,2 g MSA-MVE-Copolymer in einem 250 ml-Rundkolben mit einem KPG-Rührer (Rührblattdurchmesser: 5 cm) mit 250 Upm dispergiert. In der Meßapparatur wurde mit Hilfe einer Glaselektrode der pH-Abfall bestimmt. Als Hydrolysezeit ist jener Zeitraum in Minuten definiert, der nötig ist, um ein Absinken des pH-Wertes von pH = 12,4 auf pH = 10,0 zu erreichen. Die Hydrolysegeschwindigkeit betrug 30 Minuten.

Beispiel 2

Polymer A: Polyvinylalkohol (PVAL), Mowiol 8-88, Hoechst, BRD

Polymer B: Maleinsäureanhydrid-Methylvinylether(MSA-MVE)-Copolymer nach Beispiel 1

100 g einer 5gew.%igen wäßrigen Lösung des Polymer A wurden auf 80 °C erhitzt und unter Rühren mit 5 g des Polymers B in Pulverform versetzt. Dann wurden in einem Guß 30 ml einer 1 molaren wäßrigen Natriumhydroxidlösung zugegeben, worauf sich im Reaktionsgefäß innerhalb einer Minute ein Gel bildete. Das Gel wurde auf eine Kunststoffolie gestrichen und bei 100 °C getrocknet. 0,17 Mol-% der Säurefunktionen von Polymer B lagen verestert vor. Die Absorptionseigenschaften des getrockneten Gels wurden auf folgende Art und Weise ermittelt:

Das getrocknete Gel wurde von der Kunststoffolie abgekratzt. Etwa 0,2 g wurden in einen Teebeutel gegeben und in eine wäßrige Testlösung eingelegt. Als Testlösung wurde einerseits destilliertes Wasser, andererseits eine CIPAC-D Standardlösung, die eine wäßrige Lösung mit einem genau definierten Ionengehalt darstellt (CIPAC, Handbook, Vol. 1, Analysis of Technical Formulated Pesticides, R. Ashworth, J. Henriet, J.F. Lovett, Cellaboration International Pesticide, Analytical Council, 1970, 875 bis 879) und eine 0,9gew.%ige NaCl-Lösung verwendet. Nach 24 Stunden wurde die Teebeutel aus der Testlösung genommen, auf eine Filterpapierunterlage gelegt und solange ohne Druckanwendung gewendet, bis keine Flüssigkeit mehr austrat (ca. 4 Minuten). Anschließend wurde das Gewicht der gequollenen Probe ermittelt. Die Absorptionsfähigkeit WK des Polymerisates für eine Testlösung wurde folgendermaßen berechnet:

$$\text{WK (g/g)} = \frac{\text{Gewicht der gequollenen Probe - Gewicht des trockenen Polymers}}{\text{Gewicht des trockenen Polymers}}$$

WK-D bezeichnet die Absorptionsfähigkeit für destilliertes Wasser
WK-C bezeichnet die Absorptionsfähigkeit für eine CIPAC-D Standardlösung
WK-N bezeichnet die Absorptionsfähigkeit für eine 0,9gew.%ige Kochsalzlösung
Das Gel hatte folgende Absorptionseingeschaften:
WK-D: 434
WK-C: 49
WK-N: 49

Auf die gleiche Art und Weise wurden die Absorptionsfähigkeiten des absorbierenden Polymers auch in den weiteren Beispielen bestimmt.

## Beispiel 3

Polymer A: PVAL, Mowiol 4-88, Hoechst, BRD

Polymer B: MSA-MVE-Copolymer nach Beispiel 1

Eine 27gew.%ige wäßrige Lösung des Polymer A wurde mit einem Fluß von 7,4 g/min gemeinsam mit Polymer B in Pulverform in einer Menge von 2 g/min in ein Mischgefäß eingebracht und homogenisiert. Die entstandene Dispersion wurde kontinuierlich in einer Menge von 9,4 g/min entnommen, auf 80 °C erhitzt, mit 4 ml/min einer 17gew.%igen wäßrigen Natriumcarbonatlösung vermischt, sogleich auf eine mit 12,4 m/min laufende Papierbahn einer Breite von 23 cm aufgesprüht und mit Heißluft getrocknet. Zur Messung der Absorptionseigenschaften des absorbierenden Erzeugnisses wurden jeweils 3 bis 7 kreisförmige Proben aus der beschichteten Papierbahn gestanzt, in Testlösungen wie im Beispiel 1 beschrieben getaucht und anschließend wie im Beispiel 1 behandelt. Die Absorptionsfähigkeit des absorbierenden Erzeugnisses für eine Testlösung wurde folgendermaßen berechnet:

$$\text{WK (g/g)} = \frac{\text{Gewicht der gequollenen Probe - Gewicht des nassen Trägers}}{\text{Gewicht der trockenen Probe - Gewicht des trockenen Trägers}}$$

Das absorbierende Erzeugnis hatte folgende Absorptionseigenschaften:
WK-D: 244
WK-C: 41

Auf die gleiche Art und Weise wurde die Absorptionsfähikeit der absorbierenden Erzeugnisse in den weiteren Beispielen bestimmt.

## Beispiel 4

Polymer A: PVAL, Mowiol 4-88, Hoechst, BRD

Polymer B: MSA-MVE-Copolymer nach Beispiel 1

100 g einer 24,8gew.%igen wäßrigen Lösung von Polymer A wurden auf 80 °C erhitzt und unter Rühren mit 24,8 g des Polymers B in Pulverform versetzt. Hierauf wurden 39,4 g einer 20gew.%igen wäßrigen Natriumcarbonatlösung in einem Guß hinzugefügt. Nach etwa 30 Sekunden schäumte die Reaktionsmischung auf, wobei sich ein geschäumtes Produkt bildete, das aufgestrichen und bei 100 °C getrocknet wurde.

EP 0 366 968 A1

|        | ohne Träger | auf Papier |
|--------|-------------|------------|
| WK-D:  | 214         | 203        |
| WK-C:  | 30          | 19         |
| WK-N:  | 34          | -          |

Beispiel 5

Polymer A: PVAL, Mowiol 4-88, Hoechst, BRD

Polymer B: MSA-MVE-Copolymer nach Beispiel 1

wurde wie Beispiel 2, aber bei einer Reaktions- und Trocknungstemperatur von 25 °C ausgeführt, wobei ein getrocknetes Gel mit folgenden Eigenschaften erhalten wurde:

|        | ohne Träger | auf Papier |
|--------|-------------|------------|
| WK-D:  | 142         | 191        |
| WK-C:  | 31          | 25         |
| WK-N:  | 38          | -          |
| 0,084 Mol-% der Säurefunktionen von Polymer B lagen verestert vor. | | |

Beispiel 6

Polymer A: PVAL, Mowiol 8-88, Hoechst, BRD

Polymer B: MSA-MVE-Copolymer nach Beispiel 1

50 g einer 10gew.%igen Lösung des Polymers A wurden auf 80 °C erwärmt und unter Rühren mit 1 g des Polymers B versetzt. Hierauf wurden 30 ml einer 1 molaren wäßrigen Ammoniumhydrogencarbonatlösung in einem Guß hinzugefügt, wobei die Reaktionsmischung aufschäumte und ein geschäumtes Produkt entstand.

|        | ohne Träger | auf Papier |
|--------|-------------|------------|
| WK-D:  | 393         | 140        |
| WK-C:  | 36          | 12         |
| WK-N:  | 33          | -          |

8

Beispiel 7

Wie im Beispiel 6 beschrieben, aber unter Verwendung von 30 ml einer 0,5 molaren, wäßrigen Guanidincarbonatlösung als Base, erhielt man ein Gel.

|  | auf Papier |
|---|---|
| WK-C: | 24 |
| WK-N: | 34 |

Beispiel 8

Polymer A: Carboxymethylcellulose (CMC), Type Cekol-DVEP, Billerud, Schweden;

Polymer B: MSA-MVE-Copolymer nach Beispiel 1

100 g einer 5gew.%igen Lösung des Polymers A wurde auf 80 °C erhitzt und unter Rühren mit 5 g des Polymers B versetzt. Durch Zugabe von 30 ml einer 1 molaren wäßrigen Natriumhydroxidlösung in einem Guß entstand innerhalb einer Minute ein Gel.

|  | ohne Träger | auf Papier |
|---|---|---|
| WK-C: | 55 | 65 |
| WK-N: | 64 | - |

Beispiel 9

Polymer A: Natriumalginat, Protanal LF 20/60, AMEA, Österreich

Polymer B: MSA-MVE-Copolymer nach Beispiel 1

In 145 ml destilliertes Wasser wurden 4,5 g Polymer A und 4,5 g Polymer B eingestreut, worauf das Gemisch unter Rühren auf 70 °C erwärmt wurde. Durch Zugabe von 28,8 ml einer 1 molaren wäßrigen Natriumhydroxidlösung in einem Guß entstand innerhalb von 2 Minuten ein Gel.

|  | ohne Träger | auf Papier |
|---|---|---|
| WK-D: | 208 | 214 |
| WK-C: | 72 | 68 |

Beispiel 10

9

Polymer A: Hydroxypropylstärke, Solamyl 9570, AGENA, Österreich

Polymer B: MSA-MVE-Copolymer nach Beispiel 1

wurde wie Beispiel 9, aber unter Verwendung von 160 ml destilliertem Wasser, 12 g Polymer A, 6 g Polymer B und 53,8 ml einer 1 molaren, wäßrigen Natriumhydroxidlösung ausgeführt.

|       | ohne Träger | auf Papier |
|-------|-------------|------------|
| WK-D: | 220         | 180        |
| WK-N: | 26          | -          |

Beispiel 11

Polymer A: Kaltlösliche Stärke, Sobex 242, Südstärke, BRD

Polymer B: MSA-MVE-Copolymer nach Beispiel 1

wurde wie Beispiel 9, aber unter Verwendung von 160 ml Wasser, 6 g Polymer A, 3 g Polymer B und 26,9 ml einer 1 molaren, wäßrigen Natriumhydroxidlösung ausgeführt.

|       | ohne Träger | auf Papier |
|-------|-------------|------------|
| WK-D: | 227         | 123        |
| WK-N: | 20          | -          |

Beispiel 12

Polymer A: Phosphatguar, Meyprofilm 500, Meyhall, Schweiz

Polymer B: MSA-MVE-Copolymer nach Beispiel 1

wurde wie Beispiel 9 unter Verwendung von 160 ml Wasser, 6 g Polymer A, 6 g Polymer B und 38,4 ml einer 1 molaren, wäßrigen Natriumhydroxidlösung ausgeführt.

|       | ohne Träger | auf Papier |
|-------|-------------|------------|
| WK-D: | 152         | 215        |
| WK-C: | 18          | 28         |
| WK-N: | 22          | -          |

Beispiel 13

Polymer A: Depolymerisierter Guar, Meyprogat 90, Meyhall, Schweiz

Polymer B: MSA-MVE-Copolymer nach Beispiel 1

wurde wie Beispiel 9 unter Verwendung von 160 ml Wasser, 3 g Polymer A, 3 g Polymer B und 19,2 ml einer 1 molaren, wäßrigen Natriumhydroxidlösung angeführt.

|  | ohne Träger | auf Papier |
|---|---|---|
| WK-C: | 25 | 14 |
| WK-N: | 12 | - |

Beispiel 14

Polymer A: Nativguar, Meyproguar CSA 200/50, Meyhall, Schweiz

Polymer B: MSA-MVE-Copolymer nach Beispiel 1

wurde wie Beispiel 9 unter Verwendung von 160 ml Wasser, 3 g Polymer A, 3 g Polymer B und 26,9 ml einer 1 molaren wäßrigen Natriumhydroxidlösung ausgeführt.

|  | ohne Träger |
|---|---|
| WK-D: | 113 |
| WK-C: | 23 |
| WK-N: | 22 |

Beispiel 15

Polymer A: Hydroxypropylstärke, Solamyl 9570, AGENA, Österreich und PVAL, Mowiol 4-88, Hoechst, BRD im Verhältnis 1 : 1

Polymer B: MSA-MVE-Copolymer nach Beispiel 1

50 g einer 5gew.%igen wäßrigen Hydroxypropylstärkelösung wurde verkleistert und bei Raumtemperatur mit 12,5 g einer 20gew.%igen PVAL-Lösung versetzt und auf 80 °C erhitzt, worauf 5 g des Polymers B unter Rühren zugegeben wurden. Nach Zugabe von 30 ml einer 1 molaren wäßrigen Natriumhydroxidlösung in einem Guß entstand innerhalb einer Minute ein Gel.

|  | ohne Träger | auf Papier |
|---|---|---|
| WK-D: | 308 | 247 |
| WK-C: | 28 | 22 |
| WK-N: | 32 | - |

Beispiel 16

Polymer A: Kaltlösliche Stärke, Sobex 242, Südstärke, BRD und PVAL, Mowiol 4-88, Hoechst, BRD im Gewichtsverhältnis 1 : 1

Polymer B: MSA-MVE-Copolymer nach Beispiel 1

50 g einer 5gew.%igen wäßrigen Stärkelösung wurden mit 12,5 g einer 20 Gew.%igen wäßrigen PVAL-Lösung vermischt, auf 80 °C erwärmt und mit 5 g Polymer B in Pulverform versetzt. Nach Zugabe von 30 ml einer 1 molaren wäßrigen Kaliumhydroxidlösung in einem Guß entstand ein Gel.

|         | ohne Träger | auf Papier |
|---------|-------------|------------|
| WK-D:   | 259         | 138        |
| WK-C:   | 28          | 15         |
| WK-N:   | 27          | -          |

Beispiel 17

Polymer A: Natriumalginat, Protanal LF 20/60, AMEA, Österreich und PVAL, Mowiol 4-88, Hoechst, BRD

Polymer B: MSA-MVE-Copolymer nach Beispiel 1

100 ml einer 3gew.%igen wäßrigen Natriumalginatlösung und 7,5 g einer 20gew.%igen wäßrigen PVAL-Lösung wurden bei Raumtemperatur vermischt, auf 80 °C erhitzt und mit 4,5 g des Polymers B in Pulverform versetzt. Nach Zugabe von 27 ml einer 1 molaren wäßrigen Natriumhydroxidlösung in einem Guß entstand ein Gel.

|         | ohne Träger | auf Papier |
|---------|-------------|------------|
| WK-D:   | 250         | 220        |
| WK-C:   | 67          | 52         |

Beispiel 18

wurde wie Beispiel 17 ausgeführt, wobei das Gewichtsverhältnis der Ausgangsstoffe Natriumalginat: PVAL : MSA-MVE-Copolymer 1 : 1 : 2 betrug.

|        | ohne Träger | auf Papier |
|--------|-------------|------------|
| WK-D:  | 319         | 277        |
| WK-C:  | 52          | 50         |

Beispiel 19

wurde wie Beispiel 17 ausgeführt, wobei das Gewichtsverhältnis der Ausgangsstoffe Natriumalginat : PVAL : MSA-MVE-Copolymer 1 : 0,5 : 1,5 betrug.

|        | ohne Träger | auf Papier |
|--------|-------------|------------|
| WK-D:  | 333         | 236        |
| WK-C:  | 48          | 43         |

Beispiel 20

Polymer A: Carboxymethylcellulose (CMC), CEKOL HDEG, Billerud, Schweden und PVAL, Mowiol 4-88, Hoechst, BRD

Polymer B: MSA-MVE-Copolymer nach Beispiel 1

80 g einer 4gew.%igen wäßrigen Lösung von CMC und 16 g einer 20gew.%igen wäßrigen Lösung des PVAL wurden auf 82 °C erhitzt und mit 6,4 g Polymer B in Pulverform versetzt. Nach Zugabe von 38,4 ml einer 1 molaren wäßrigen Natriumhydroxidlösung in einem Guß entstand innerhalb 1 Minute ein Gel.

|        | ohne Träger | auf Papier |
|--------|-------------|------------|
| WK-D:  | 289         | 383        |
| WK-C:  | 64          | 39         |
| WK-N:  | 55          | -          |

Beispiel 21

Polymer A: Hydrolysierte Weizenstärke, Merigum C, Amylum, Belgien und PVAL, Mowiol 8-88, Hoechst, BRD

Polymer B: MSA-MVE-Copolymer nach Beispiel 1

6 g Weizenstärke und 6 g PVAL wurden in 123 ml destilliertem Wasser bei 82 °C verkleistert bzw. gelöst, worauf 6 g MSA-MVE-Copolymer dispergiert in 20 ml Wasser unter Rühren zugefügt wurden. Nach Zugabe von 39 ml einer 1 molaren wäßrigen Natriumhydroxidlösung in einem Guß entstand innerhalb von 30 bis 50 Sekunden ein Gel.

13

|       | ohne Träger | auf Papier |
|-------|-------------|------------|
| WK-D: | 374         | 188        |
| WK-C: | 18          | 16         |
| WK-N: | 18          | -          |

Beispiel 22

Polymer A: Kationische Kartoffelstärke, Cationamyl 9852, AGENA, Österreich und Xanthan Gum, Jungbunzlauer, Österreich

Polymer B: MSA-MVE-Copolymer nach Beispiel 1

6 g Kartoffelstärke und 3 g Xanthan Gum wurden in 140 ml destilliertem Wasser bei 80 °C verkleistert bzw. gelöst und mit 3 g Polymer B, das in 20 ml Wasser dispergiert worden war, versetzt. Nach Zugabe von 27 ml einer 1 molaren wäßrigen Natriumhydroxidlösung in einem Guß entstand innerhalb von 30 Sekunden ein Gel.

|       | ohne Träger | auf Papier |
|-------|-------------|------------|
| WK-D: | 144         | 113        |
| WK-C: | 19          | 25         |
| WK-N: | 17          | -          |

Beispiele 23 - 27

3 g verschiedener zerkleinerter Zellstoffmaterialien wurden in 100 g einer 5gew.%igen PVAL-Lösung (Mowiol 8-88, Hoechst, BRD) eingebracht und unter Rühren fein zerfasert. Diese Mischung wurde auf 80 °C erhitzt und mit 8 g MSA-MVE-Copolymer nach Beispiel 1 versetzt. Nach Zugabe von 48 ml einer 1 molaren wäßrigen Natriumhydroxidlösung in einem Guß entstand innerhalb von 20 Sekunden ein Gel.

Beispiel 23

Zellstoffmaterial: Photozellstoff, Borregaard, Österreich
PVAL : Zellstoff : MSA-MVE-Copolymer = 1 : 0,6 : 1,6 (5 g : 3 g : 8 g)
48 ml 1 molare HaOH

|       | ohne Träger | auf Papier |
|-------|-------------|------------|
| WK-D: | 334         | 254        |
| WK-C: | 24          | 23         |
| WK-N: | 18          | -          |

Beispiel 24

14

Zellstoffmaterial: Sulfatzellstoff, Mahlgrad 12° SR, Weiße 88,7 %, Pöls, Österreich
PVAL : Zellstoff : MSA-MVE-Copolymer = 1 : 0,6 : 1,6 (5 g : 3 g : 8 g)
48 ml 1 molare NaOH

|        | ohne Träger | auf Papier |
|--------|-------------|------------|
| WK-D:  | 390         | 256        |
| WK-C:  | 25          | 19         |
| WK-N:  | 18          | -          |

## Beispiel 25

Zellstoffmaterial: Küchenrolle A, Zewa, PWA, BRD
PVAL : Zellstoff : MSA-MVE-Copolymer = 1 : 0,6 : 1,6 (5 g : 3 g : 8 g)
48 ml 1 molare NaOH

|        | ohne Träger | auf Papier |
|--------|-------------|------------|
| WK-D:  | 284         | 251        |
| WK-C:  | 29          | 27         |
| WK-N:  | 22          | -          |

## Beispiel 26

Zellstoffmaterial: Küchenrolle B, Henry, Laakirchen, Österreich
PVAL : Zellstoff : MSA-MVE-Copolymer = 1 : 1 : 2 (5 g : 5 g : 10 g)
60 ml 1 molare NaOH

|        | ohne Träger | auf Papier |
|--------|-------------|------------|
| WK-D:  | 227         | 196        |
| WK-C:  | 23          | 28         |
| WK-N:  | 23          | -          |

## Beispiel 27

Zellstoffmaterial: Sulfitzellstoff, vollgebleicht, Steyrermühl, Österreich
PVAL : Zellstoff : MSA-Copolymer = 1 : 1 : 2 (5 g : 5 g : 10 g)
60 ml 1 molare NaOH

|  | ohne Träger | auf Papier |
|---|---|---|
| WK-D: | 218 | 204 |
| WK-C: | 20 | 18 |
| WK-N: | 17 | - |
| 0,113 Mol-% der Säurefunktionen von Polymer B lagen verestert vor. | | |

## Beispiel 28

PVAL-Bernsteinsäureester

100 g einer 36 Gew.%igen, wäßrigen Lösung von PVAL (Mowiol 4-88, Hoechst, BRD) wurden mit 24,5 g Bernsteinsäureanhydrid und 0,3 g konzentrierter Schwefelsäure bei 60 °C 1 Stunde gerührt. Anschließend wurde das Reaktionsgemisch auf Raumtemperatur abgekühlt und in Aceton eingetropft, wobei der PVAL-Bernsteinsäureester ausgefällt wurde. Der Niederschlag wurde abfiltiert, mit Aceton gewaschen und bei 50 °C bis zur Gewichtskonstanz getrocknet. Dabei wurden 45 g PVAL-Bernsteinsäureester erhalten. Die Bestimmung des Substitutionsgrades DS erfolgte tritrimetrisch. Der Substitutionsgrad betrug 0,149 Mol Bernsteinsäureestergruppen pro Mol Hydroxylgruppen im PVAL.

Nach der obigen Verfahrensweise unter Einsatz entsprechender Mengen Bernsteinsäureanhydrid wurden PVAL-Bernsteinsäureester mit Substitutionsgraden von 0,016; 0,038; 0,091 und 0,293 Mol Bernsteinsäureestergruppen pro Mol Hydroxylgruppen im PVAL hergestellt.

Nach der im Beispiel 2 angegebenen Verfahrensweise unter Verwendung von jeweils 5 g PVAL-Bernsteinsäureester mit den entsprechenden Substitionsgraden DS, 5 g Polymer B und 30 ml 1 molaren wäßrigen Natriumhydroxidlösung wurden die Verbindungen 29 bis 33 hergestellt:

## Beispiele 29 - 33

Polymer A: PVAL-Bernsteinsäureester mit Substitutionsgrad DS

Polymer B: MSA-MVE-Copolymer nach Beispiel 1

| Nr. | DS | WK-D ohne Träger | WK-C ohne Träger | WK-N ohne Träger |
|---|---|---|---|---|
| 29 | 0,016 | 431 | 54 | 50 |
| 30 | 0,038 | 398 | 61 | 52 |
| 31 | 0,091 | 353 | 64 | 48 |
| 32 | 0,149 | 400 | 64 | 41 |
| 33 | 0,293 | 424 | 61 | 43 |

## Beispiel 34

PVAL-Hydroxypropansulfonsäureether

100 g einer 20 gew.%igen, wäßrigen Lösung von PVAL, Mowiol 8-88, Hoechst, BRD wurden mit 17,8 g einer 25gew.%igen, wäßrigen Lösung von 3-Chlor-2-hydroxypropansulfonsäure-Na (CHPS-Na) versetzt und auf 60 °C erwärmt. Der pH-Wert wurde dabei durch tropfenweise Zugabe einer 20gew.%igen, wäßrigen Natriumhydroxidlösung auf pH 8,0 gehalten. Nach beendeter Reaktion wurde die Reaktionslösung in Aceton eingetropft, wobei der PVAL-Hydroxypropansulfonsäureether ausgefällt wurde. Der Niederschlag wurde abfiltriert, mit Aceton gewaschen und getrocknet. Dabei wurden 21 g PVAL-Hydroxypropansulfonsäureether gewonnen.

Nach der obigen Verfahrensweise wurden unter Einsatz entsprechender Mengen an 3-Chlor-2-hydroxypropansulfonsäureether PVAL-Hydroxypropansulfonsäureether hergestellt.

Nach der im Beispiel 2 beschriebenen Verfahrensweise unter Einsatz von jeweils 20 g an PVAL-Hydroxypropansulfonsäureether als Polymer A gelöst in 80 ml destilliertem Wasser, jeweils 20 g MSA-MVE-Copolymer hergestellt nach Beispiel 1 als Polymer B und 120 ml einer 1 molaren wäßrigen Natriumhydroxidlösung wurden die folgenden Polymerisate hergestellt:

| Beispiel 35 - 39 | | | | | | |
|---|---|---|---|---|---|---|
| Nr. | Menge an CHPS-Na (25gew.%) in g | WK-D | | WK-C | | WK-N |
| | | ohne Träger | auf Papier | ohne Träger | auf Papier | ohne Träger |
| 35 | 3,57 | 423 | 347 | 37 | 36 | 34 |
| 36 | 17,84 | 462 | 293 | 47 | 30 | 50 |
| 37 | 39,59 | 326 | 245 | 28 | 20 | 28 |
| 38 | 107,08 | 268 | 398 | 34 | 24 | 43 |
| 39 | 278,48 | 337 | 358 | 34 | 26 | 35 |

## Beispiel 40

PVAL-2-Hydroxypropan-3-(trimethylammoniumchlorid)-ether

In 200 ml einer 5gew.%igen, wäßrigen Lösung von PVAL, Mowiol 8-88, Hoechst, BRD nach Beispiel 1 wurden bei Raumtemperatur 0,40 g Glycidyltrimethylammoniumchlorid eingerührt, die Reaktionsmischung auf 60 °C erwärmt und durch kontinuierliche Zugabe einer wäßrigen Natriumhydroxidlösung auf pH 8 gehalten. Nach 2 Stunden wurde das Reaktionsgemisch auf Raumtemperatur abgekühlt, das gebildete Produkt durch Zugabe von Aceton ausgefällt, abfiltriert, gewaschen und bei 50 °C getrocknet.

Dabei wurden 10,4 g der Titelverbindung erhalten.

## Beispiel 41

Nach der im Beispiel 2 beschriebenen Verfahrensweise unter Verwendung von 5 g des Produktes aus Beispiel 40, gelöst in 100 ml destilliertem Wasser, 5 g MSA-MVE-Copolymer hergestellt nach Beispiel 1 in Pulverform und 30 ml einer 1 molaren wäßrigen Natriumhydroxidlösung ein Produkt mit folgenden Eigenschaften erhalten:

| | ohne Träger |
|---|---|
| WK-D: | 353 |
| WK-C: | 35 |
| WK-N: | 51 |

17

Beispiel 42

6 g Kationische Kartoffelstärke (Amylofax 15, DS = 0,027, AVEBE, NL) und 6 g PVAL (Mowiol 8-88, Hoechst, BRD) wurden in 123 ml Wasser bei 82 °C verkleistert bzw. gelöst und mit 6 g MSA-MVE-Copolymer hergestellt nach Beispiel 1, dispergierten 20 ml Wasser versetzt. Nach Zugabe von 39 ml einer 1 molaren wäßrigen Natriumhydroxidlösung in einem Guß entstand innerhalb von Sekunden ein Gel.

|  | ohne Träger | auf Papier |
|---|---|---|
| WK-D: | 253 | 159 |
| WK-C: | 18 | 16 |
| WK-N: | 16 | - |

Beispiel 43

6 g Kationische Kartoffelstärke (Amylofax 15, DS = 0,027, AVEBE, NL) und 3 g CMC (CEKOL HDEG, Billerud, Schweden) wurden bei 80 °C in 140 ml destilliertem Wasser verkleistert bzw. gelöst und mit einer wäßrigen Dispersion von 3 g MSA-MVE-Copolymer, hergestellt nach Beispiel 1 in 20 ml Wasser versetzt. Nach Zugabe von 27 ml einer 1 molaren wäßrigen Natriumhydroxidlösung in einem Guß entstand innerhalb weniger Sekunden ein Gel.

|  | ohne Träger | auf Papier |
|---|---|---|
| WK-D: | 230 | 156 |
| WK-C: | 32 | 30 |

Beispiel 44

Polymer A: Kationische Kartoffelstärke, Cationamyl 9852, AGENA, Österreich

Polymer B: MSA-Isobutylen-Copolymer, Isobam-10, Kuraray, Japan

6 g Polymer A wurden bei 70 °C in 160 ml destilliertem Wassr verkleistert und mit 3 g Polymer B, das vorher in einer Reibschale vermahlen worden war, unter Rühren versetzt. Nach Zugabe von 26,5 ml einer 1 molaren wäßrigen Ammoniumhydroxidlösung in einem Guß, entstand ein Gel.

|  | ohne Träger |
|---|---|
| WK-D: | 127 |

Die folgenden Beispiele 45 bis 48 beweisen, daß das erfindungsgemäße Polymerisat über Esterbindungen und nicht nur unter Wasserstoffbindungen vernetzt ist.

Beispiel 45

1 g kationische Kartoffelstärke, Cationamyl 9852, AGENA, Österreich wurden in 150,6 g destilliertem Wasser bei 70 °C 20 Minuten verkleistert und mit 0,5 MSA-MVE-Copolymer, hergestellt nach Beispiel 1, dispergiert in 9,5 ml destilliertem Wasser, versetzt und 30 Sekunden gerührt, worauf 3,84 ml einer 1 molaren wäßrigen Natriumhydroxidlösung in einem Guß zugegeben wurden, wobei ein Gel entstand. Der Feststoffgehalt der Reaktionsmischung

$$\frac{(\text{eingesetzte Feststoffe in g } \times 100)}{\text{Gesamtgewicht in g}}$$

betrug dabei 1 %.Nach Abkühlen auf Raumtemperatur wurde die Viskosität der Reaktionsmischung bestimmt.

Auf gleiche Weise wurden unter Verwendung der entsprechenden Menge an Ausgangsstoffen Reaktionsgemische mit Feststoffgehalten von 2 % und 3 % hergestellt.

Zur Vergleich wurden auf gleiche Weise, aber unter Verwendung eines MSA-MVE-Copolymers, hergestellt nach Beispiel 1, in dem vor Zugabe zur Reaktiosmischung durch 14-stündiges Rühren in destilliertem Wasser die Säureanhydridgruppen vollständig verseift waren, Reaktionsgemische mit Feststoffgehalten von 1, 2 und 3 % hergestellt und deren Viskositäten bestimmt. In allen Fällen betrug der Neutralisationsgrad 60 %, d.h. 60 % Säuregruppen im Polymerisat lagen als Na-Salz vor, und der pH = 7,5.

Die Viskosität wurde mit Hilfe eines Brookfield Viskosimeter, Synchro Lectric Viscometer, Model LVT bestimmt. Folgende Viskositäten wurden gemessen:

| Feststoffgehalt in Prozent | Viskosität in mPas | |
|---|---|---|
| | erfindungsgemäß | Vergleich |
| 1 | 520 | 120 |
| 2 | 8350 | 525 |
| 3 | 339000 | 2190 |

Beispiel 46

2 g Polyvinylalkohol, Mowiol 8-88, Hoechst, BRD wurden bei 80 °C in 89,1 g destilliertem Wasser gelöst und mit 1 g MSA-MVE-Copolymer, hergestellt nach Beispiel 1, dispergiert in 9 ml Wasser, versetzt und 30 Sekunden gerührt, worauf 3,2 ml einer 1 molaren wäßrigen Natriumhydroxidlösung in einem Guß zugegeben wurden, wobei ein Gel entstand. Der Feststoffgehalt der Reaktionsmischung betrug 3 %. Nach Abkühlung auf Raumtemperatur wurde die Viskosität der Reaktionsmischung bestimmt.

Auf gleiche Weise wurden unter Verwendung der entsprechenden Menge an Ausgangsstoffen Reaktionsgemische mit Feststoffgehalten von 5 % und 7 % hergestellt und deren Viskositäten gemessen.

Zum Vergleich wurde auf gleiche Weise, aber unter Verwendung eines MSA-MVE-Copolymers, hergestellt nach Beispiel 1, in dem vor der Zugabe zur Reaktionsmischung durch 14-stündiges Rühren in destilliertem Wasser die Säureanhydridgruppen vollständig geöffnet waren, Reaktionsmischungen hergestellt, und deren Viskositäten bestimmt. In allen Fällen betrug der Neutralisationsgrad 25 % und der pH = 4,05.

EP 0 366 968 A1

| Feststoffgehalt in Prozent | Viskosität in mPas | |
|---|---|---|
| | erfindungsgemäß | Vergleich |
| 3 | 1660 | 158 |
| 5 | 7945 | 550 |
| 7 | 36310 | 1740 |

**Ansprüche**

1. Absorbierendes Polymerisat, bestehend aus einem Polymer A und einem Polymer B, dadurch gekennzeichnet, daß das Polymer A zu einem Anteil von 10 bis 99 Gewichtsprozent vorliegt und ein gegebenenfalls modifizierter Polyvinylalkohol oder ein gegebenenfalls modifiziertes Polysaccharid oder eine Mischung derselben ist und das Polymer B zu einem Anteil von 1 bis 90 Gewichtsprozent vorliegt und ein Maleinsäureanhydrid-Homo- oder Copolymer mit einer Hydrolysezeit von 5 bis 120 Minuten ist, wobei die Anhydridgruppen geöffnet und 0,005 bis 5 Mol-% der Säurefunktionen im Polymer B mit Hydroxygruppen des Polymers A verestert sind und die übrigen Säurefunktionen als freie Säuregruppen, als Salze oder als Säureamidgruppen vorliegen.

2. Absorbierendes Polymerisat nach Anspruch 1, dadurch gekennzeichnet, daß Polymer A ein modifizierter Polyvinylalkohol ist.

3. Absorbierendes Polymerisat nach Anspruch 1, dadurch gekennzeichnet, daß Polymer A Carboxymethylcellulose ist.

4. Absorbierendes Polymerisat nach Anspruch 1, dadurch gekennzeichnet, daß Polymer B ein Maleinsäureanhydrid-Methylvinylether-Copolymer ist.

5. Absorbierendes Polymerisat nach den Ansprüchen 1 bis 4, dadurch gekennzeichnet, daß die Hydrolysezeit von Polymer B 10 bis 60 Minuten beträgt.

6. Absorbierendes Polymerisat nach einem der Ansprüche 1 bis 5, dadurch gekennzeichnet, daß Polymer A in einem Anteil von 40 bis 95 Gewichtsprozent und Polymer B in einem Anteil von 5 bis 60 Gewichtsprozent vorliegt.

7. Verfahren zur Herstellung eines absorbierenden Polymerisates, dadurch gekennzeichnet, daß man einen gegebenenfalls modifizierten Polyvinylalkohol, ein gegebenenfalls modifiziertes Polysaccharid oder Mischungen derselben mit einem Maleinsäureanhydrid-Homo- oder Copolymer mit einer Hydrolysezeit von 5 bis 120 Minuten vereinigt und bei einer Temperatur von 20 bis 120 °C in einem Guß mit 0,5 bis 1,6 Äquivalenten einer anorganischen oder organischen Base pro Äquivalent Maleinsäureanhydrid versetzt.

8. Absorbierendes Erzeugnis, dadurch gekennzeichnet, daß das absorbierende Polymerisat nach Anspruch 1 mit einem oder mehreren Trägern verbunden ist.

9. Absorbierendes Erzeugnis nach Anspruch 8, dadurch gekennzeichnet, daß der oder die Träger eine oder mehrere Papierbahnen sind.

10. Verfahren zur Herstellung eines absorbierenden Erzeugnisses nach Anspruch 8, dadurch gekennzeichnet, daß ein Polymer A, ein Polymer B und eine wäßrige Lösung einer anorganischen oder organischen Base gegebenenfalls unter Erhitzen auf und/oder zwischen einen oder mehrere Träger aufgebracht und getrocknet werden.

20

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | US-A-4 169 818 (DE MARTINO) * Seite 7, Zeilen 44-68; Seite 3, Zeilen 49-68 * --- | 1,4,7 | C 08 L 35/00 C 08 L 29/02 C 08 L 1/00 C 08 L 3/00 C 08 L 5/00 A 61 L 15/24 A 61 L 15/28 A 61 L 15/60 |
| A | JOURNAL OF APPLIED POLYMER SCIENCE, Band 23, 1979, Seiten 2611-2625; GRYTE et al.: "Hydrophilic Interpolymer Membranes from Crosslinked Blends of Poly (vinylalcohol), Poly(styrene sodium sulfonate) and Poly(vinyl methyl ether-alt-maleic anhydride) * Seite 2612, Zeilen 16-20; Seite 2613, Zeilen 9-21 * ----- | 7 | |

**RECHERCHIERTE SACHGEBIETE (Int. Cl.5)**

C 08 L
A 61 L
C 08 G

Der vorliegende Recherchenbericht wurde für alle Patentansprüche erstellt

| Recherchenort | Abschlußdatum der Recherche | Prüfer |
|---|---|---|
| DEN HAAG | 25-01-1990 | WILSON A.J.D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes Dokument

EPO FORM 1503 03.82 (P0403)